# EUROPEAN PATENT APPLICATION

(11) **EP 2 912 998 A1**
(43) Date of publication of application: **02.09.2015**
(21) Application number: 14156881.6
(22) Date of filing: 26.02.2014
(51) Int. Cl.: A61B 5/05, A61B 5/055

(54) **A tissue anomaly detection apparatus**

(71) Applicant: Spagnolini, Umberto, 20125 Milan (IT); Bellorofonte, Carlo, 20157 Milan (IT); Canonico, Michelangelo, 20149 Milan (IT)
(72) Inventor: Spagnolini, Umberto, 20125 Milan (IT); Bellorofonte, Carlo, 20157 Milan (IT); Canonico, Michelangelo, 20149 Milan (IT)
(74) Representative: Postiglione, Ferruccio

(57) **Abstract**

A tissue anomaly detection apparatus (1000), comprising: a patient positioning structure (1) configured to allow the patient assuming a detection position; a probe antenna device (2) structured to radiate incident radio-frequency signal to irradiate a patient tissue in near-field conditions so as to produce a first resulting radio-frequency signal (S_{BS}). The detection apparatus is characterised in that it further includes: a reflector (3) positioned so as to reflect at least part of the incident radio-frequency signal which has not irradiated the patient tissue and generate a reflected signal (S_{R}), a receiving device (4) structured to receive the first resulting signal and the reflected signal and provide corresponding received data, a processing module (5) structured to process the received data to provide an information indicating detection of an anomaly in said tissue.

## Description

### Technical field

The present invention relates to electromagnetic detection systems for the detection of anomalies (e.g. tumors and calcifications) in living tissue, and in particular to early detection of breast, colorectal and prostatic cancer.

### Background of the invention

Document EP-A-2465428 discloses a tissue anomaly detection method employing a probe antenna device, i.e. a transmitter equipped with one or two antennas coupled in the near field conditions with tissues. The probe antenna device described in this prior art document is moved by a clinician for irradiating a tissue of a patient in near-field conditions so as to produce a radio-frequency signal resulting from the interaction with the aforementioned tissue. The system also comprises a receiving device for receiving said resulting signal and a processing module structured to process the received data in order to provide an information indicating the detection of an anomaly in said tissue. In particular, the probe antenna device has a fixed radiation frequency range and it is structured so that the resulting signal is a scattered signal resulting from a combination of the incident signal and an induced radio-frequency signal resulting from the coupled tissue.

According the description of EP-A-2465428 the scattered signal is received and electronically analysed to detect an anomaly associated with nulls or minima in the pattern of the scattered electrical field power versus frequency and receiving antenna position.

### Summary of invention

The Applicant has observed that in the known anomaly diagnosis apparatuses an improvement of the detectability of the anomalies is desirable. As an example, with reference to the above described prior art technique, the Applicant observes that there is a need of enhancing resolution and stability of null and minima in the backscattered pattern.

The Applicant has found that an enhancing of the anomalies detectability can be obtained with a tissue anomaly detection apparatus as described by the attached claim 1. Particular embodiments of the apparatus are described by the dependent claims 2-15.

### Brief description of the drawings

Further characteristics and advantages will be more apparent from the following description of preferred embodiments given as a way of an example with reference to the enclosed drawings in which:
- figure 1 schematically shows a preferred embodiment of a tissue anomaly detection apparatus;
- figure 2 shows an embodiment of a probe antenna device employable in said detection apparatus;
- figure 3A and figure 3B show two different particular supporting structure shapes for an array of receiving antennas employable in said detection apparatus.

### Detailed description

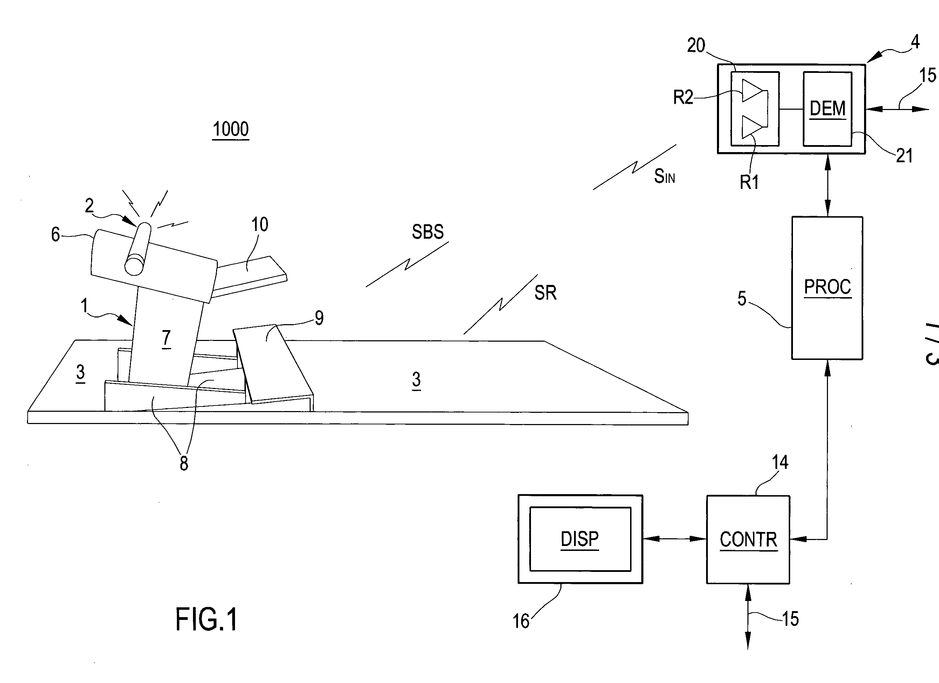

Figure 1 shows an embodiment of a tissue anomaly detection apparatus 1000 which can be used to detect anomalies in tissues of human or animal bodies. The anomalies can be, as an example, tumors and/or calcifications. Particularly, tumors in early stage (e.g. malignant breast, prostatic and colorectal tumors) can be detected by the detection apparatus 1000.

Particularly, the detection apparatus 1000 can implement a detection methodology via a probe antenna device according to the document EP-A-2465428.

The detection apparatus 1000 comprises: a patient positioning structure 1 configured to allow the patient assuming a detection position, a probe antenna device 2, a reflector 3, a receiving device 4 and a processing module 5 (PROC). Moreover, according to a particular embodiment, the detection apparatus 1000 comprises a controller module 14 (CONTR) connected, via a wired or wireless link 15, to the probe antenna device 2 and also connected to the processing unit 5, and a display 16 (DISP).

The patient positioning structure 1 can be a support frame designed to support the patient or one of her/his body part. The example shown in figure 1 refers to a detection apparatus 1000 particularly adapted to the diagnosis of prostatic cancer.

In accordance with this example, the patient positioning structure 1 comprises seat means 6 adapted to accommodate a patient according to the anatomy of said patient. Particularly, the seat means 6 shown in figure 1 comprises a saddle shaped support on which said patient is adapted to sit astride and a one more leg elements 7 fixed to the saddle shaped support 6 and to a base structure 8. Preferably, the patient positioning structure 1 can be provided with a footrest element 9 in form of a dais and an optional support aid element 10 useful for patient stability when sitting.

The detection apparatus 1000 is also provided with a supporting frame configured to support the probe antenna device 2. In accordance with the shown example, the probe antenna device 2 is supported by the saddle shaped support 6 which is provided with a cavity in which the probe antenna device 2 is placed. In this case, the patient sits on the probe antenna device 2 mechanically coupled to the saddle shaped support 6, and put his/her feet on the footrest element 9 with legs widely open so as to be in close contact with the an external portion of probe antenna device 2.

The probe antenna device 2 is structured to radiate a narrowband incident radio-frequency signal to irradiate a patient tissue in near-field conditions.

To the purpose of the present description, a narrowband signal shows a bandwidth Bw which is small enough to use the assumption that a response as result of the interaction with body can be considered constant within the bandwidth Bw, provided that 1/Bw is below the relaxation times of the irradiated patient biological tissues (typical value Bw>1KHz).

The probe antenna device 2 generates an electromagnetic field (i.e. an incident signal) that locally couples in near-field with the tissues surrounding the contact point. As an example, with reference to the prostatic tumor it is observed that around the perineum the area is electromagnetically very heterogeneous and it cannot be described by just simple macroscopic relative permittivity values. However, for the sake of reasoning, by assuming a relative permittivity at 434 MHz that ranges between 7 and 12 as for mixed fat and bones, the wavelength in the body is about 19-25 cm. Prostate size is 3-4cm and it is embedded within 3-4cm from the perineum position where the probe antenna device 2 is placed when patient is sitting on saddle shaped support 6 with legs spread to favour by the weight of the patient's body the minimum distance between prostate and antenna device 2. Overall, the coupling is in the near-field condition.

More specifically, "near-field" coupling means that the distance between the radiating antenna (e.g. the probe antenna device 2) and the target is shorter than the wavelength assumed by the radiation in the patient body. Particularly, this distance can be lower than 1/5 of the wavelength, preferably lower than 1/10 of the wavelength assumed in the body or even less As an example, in prostate inspection the distance between the radiating antenna (e.g. the probe antenna device 2) and the target is included in the range 3/25 - 4/19 of the wavelength evaluated in the patient body. Near-field operation enables a coupling with malignant tissues that is much higher than those normally achievable by conventional far-field systems used for microwave imaging.

The cancerous cells in depth tissues of the tumor region are excited from radio-frequency, that in turn generates a secondary electromagnetic field (i.e. an induced signal) for the large permittivity values of tumor tissues. The incident electromagnetic field and the induced electromagnetic field generate a scattered electromagnetic field (scattered signal). Particularly, since the tumor is in close distance from the probe antenna device 2 in term of wavelength, the scattered field is a complex combination of incident field and the one generated by heterogeneous tissues with tumor (if present).

The closer is the probe antenna device 2 to the tumor embedded into heterogeneous tissues, and the stronger is the coupling with the tumor, this in turn generates a more complex backscattering pattern with multiple scattering nulls.

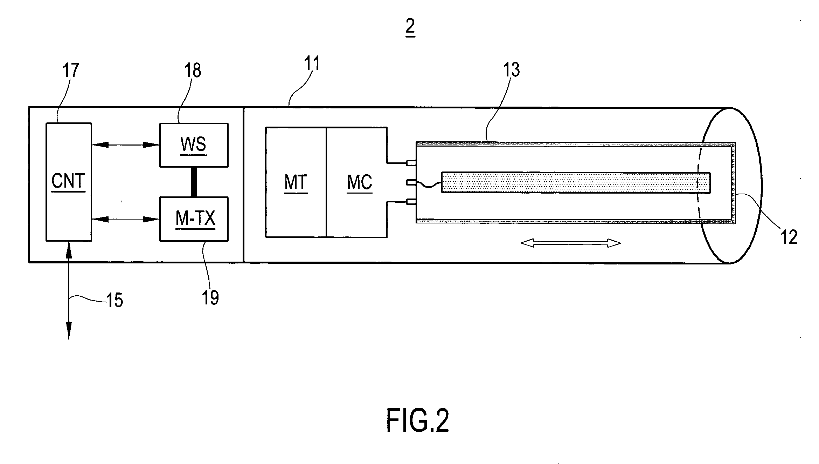

A particular embodiment of the probe antenna device 2 is now described with reference to figure 2. The probe antenna device 2 comprises an external case 11 housing at least one antenna radiating element 12 and a motor MT mechanically coupled with the antenna radiating element 12 to cause a sliding motion of the antenna radiating element 12 inside the external case 11. The external case 11 is electromagnetically transparent to avoid any remarkable interaction with the waves from the antenna radiating element 12, or it is dielectric with permittivity comparable to the radiating element 12, or with permittivity comparable with the normal tissues. There are many possible solutions to move the radiating element 12 with respect to the external case 11. As an example, the motor MT (e.g. a step or a brushless motor) can be coupled to the antenna radiating element 12 by a mechanical coupling module MC that can be an endless screw or a rack-and-pinion mechanism in dielectric material to avoid any possible interaction with the field radiated by the antenna radiating element 12 and coupled with a patient tissue. The antenna radiating element 12 is sliding movable in the external case 11 between a retracted position and extended position. The movement of the antenna radiating element 12 allows to scan the investigating region of the patient body. In the example of prostate tumor detection, it translates in the low buttocks and perineum area in the range of 10-80cm. For breast cancer detection, the movement area of the antenna radiating element 12 is all the region around breast till the armpit.

Particularly, the antenna radiating element 12 produces a non-ionizing radiofrequency radiation. Moreover, antenna radiating element 12 can operate to generate a linearly polarized electromagnetic field and can be an isotropic irradiator specifically designed to maximally couple with the tissues in near field.

In accordance with a particular example, the antenna radiating element 12 is further housed in an internal case 13 made, as an example, of dielectric material such as plastic, and analogous to the external case 11. It is also observed that the patient positioning structure 1 is advantageously made of dielectric materials with minimal (ideally, null) dielectric contrast with respect of the external case 11 and the internal case 13 of the probe antenna device 2. As an example, the patient positioning structure 1 is made of the same material of the external case 11 and the internal case 13.

The probe antenna device 2 can be also provided with a probe controller 17 (CNT), an electromagnetic wave source 18 (WS) and a transmitter modulator 19 (M-TX), schematically represented in figure 2.

The electromagnetic wave source 18 can be a variable frequency oscillator controlled by the probe controller 17 and it is connected to the transmitter modulator 19 (M-TX). As an example, the electromagnetic wave source 18 generates a radiation having frequency included into the range 1 MHz - 1000 MHz and particularly, 1 MHz - 900 MHz and more particularly, 300 MHz - 700 MHz.

According to an example, the electromagnetic wave source 18 is configured to generate radio-frequency signals at K frequencies f₁,f₂,...,f_{K} properly chosen to be within a predefined frequency span around a central carrier frequency f₀ with 350 MHz <f₀< 550 MHz. The maximum frequency span of the K frequencies might be below 100MHz, typical values are 1 MHz, 5MHz, 10MHz. Particularly, the number of frequencies can be K=201, even K=31, K=7 and degenerate to K=1 in some cases.

Moreover, the transmitter modulator 19 can include a signature generation block structured to generate a modulated signal vector. The signatures are used to distinguish the dual polarizations when the radiating element 12 is an antenna with dual polarization, and can enhance the diagnostic properties of the received signals. In accordance with the example in which the number K is different from 1, the transmitter modulator 19 is also provided with a FDM (Frequency Division Multiplexing) block to multiplex each of said radio-frequency signals at K frequencies f₁, f₂,..., f_{K}.

It is observed that even if the following description refers to the embodiment in which the detection apparatus 1000 operates with electromagnetic waves at a frequency below 700 MHz and particularly in the frequency range 350MHz <f₀< 550MHz, the detection apparatus 1000 can operates at a different frequency range, e.g.: the central carrier frequency f₀ can be in the range equal to 800 MHz - 1000 MHz. Particularly, the central carrier frequency f₀ can be in the range equal to 850 MHz - 980 MHz.

Particularly, the signal generation technique employed by the probe antenna device 2 can be analogous to one of the techniques described in the already cited document EP-A-2465428.

Moreover, the probe antenna device 2 may be provided with a variable-gain amplifier placed between the modulating module 19 and the antenna radiating element 12 (not shown).

Particularly, the probe controller 17 exchanges data and command signals with the controller module 14 and controls the electromagnetic wave source 18, the transmitter modulator 19 and the motor MT. The transmitter modulator 19 can be connected to the antenna radiating element 12 by means of a suitable cable (not shown).

It is also observed that in accordance with another example the electromagnetic wave source 18, the probe controller 17 and the transmitter modulator 19 can be external to the probe antenna device 2.

Furthermore, the motor MT and mechanical coupling module MC that guarantee the movements of the antenna radiating element 12 can be external to the external case 11 with pulleys and gear racks that transfer the movements to the radiating antenna element 12.

Reference is now made to the reflector 3 which is positioned so as to reflect at least part of the incident radio-frequency signal produced by the probe antenna device 2, preferably not irradiated to the patient tissue, so as providing a reflected reference signal for interferometry measurements.

Particularly, the reflector 3 is positioned in such a way to avoid that it shields or obstructs the line of sight propagation from the probe antenna device 2 to the receiving device 4.

With reference to the carrier frequencies of the incident signal radiated by the antenna probe device 2, the reflector 3 shows a reflectivity R whose amplitude is in the range between 0.6 and 1.

The reflector 3 can be, as an example, a flat plate and can be made of perfect conducting material (e.g. copper or aluminum), or liquids (water with dissolved salts at different concentration, e.g. NaCl) embedded into a two-layer containment structure (e.g. plexiglas) or any mixture of materials as known in the art [L.Taylor, IEEE Trans. On Antennas and Propagation, 1965]. All these solutions are used to reach the predefined reflectivity R.

In accordance with the embodiment of figure 1 the reflector 3 is positioned, for example on the floor, under the patient positioning structure 1 so as that the probe antenna device 2 is placed between the sited patient and the reflector 3 to generate a reflected reference signal S_{R}. However, the size of the reflector 3 can be smaller than the one shown in figure 1. Particularly, the size of the reflector 3 can be in the order of one wavelength for central carrier frequency f₀, provided that the reflector 3 is placed halfway between the probe antenna device 2 and the receiving device 4.

The reflector 3 has predefined electromagnetic properties that enhance a backscattered wave field when the antenna probe device 2 couples with pathological tissues.

The receiving device 4 can be analogous to the "receiving device 200" described in the EP-A-2465428. As an example, the receiving device 4 is provided with an array 20 comprising at least two receiving antennas R1 and R2, schematically shown in figure 1. The two antennas R1 and R2 are, as an example, dipoles on printed circuits. As another example, the array 20 can include four sub-arrays each comprising eight pair of antennas as R1 and R2 in figure 1.

The array 20 allows to provide an electrical signal corresponding to a resulting electromagnetic signal obtained by the incident electromagnetic signal as backscattered by the irradiated patient tissue and the electromagnetic signal reflected by the reflector 3.

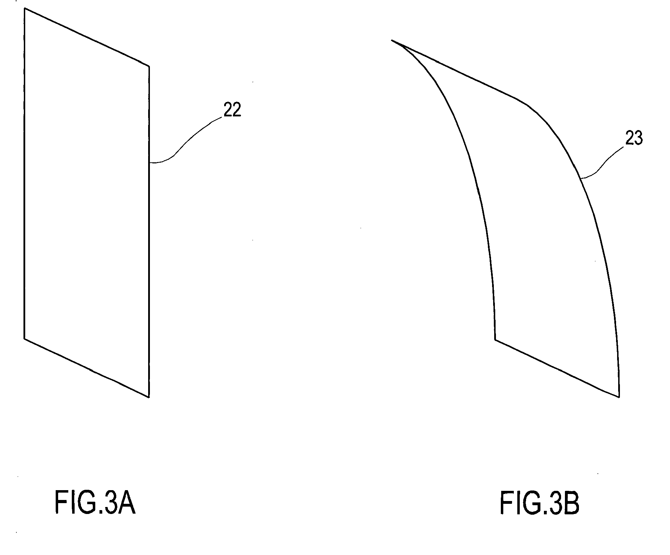

The multiple antennas included in the array 20 can be arranged on a vertical flat support plane 22 as shown in figure 3A (e.g. orthogonal to the plane of the reflector 3 as shown in figure 1). According to another embodiment, the antennas included in the array 20 can be arranged on a curved support plate 23 as shown in figure 3b which has a concavity facing the probe antenna 2. The curvature of the curved support plate 23 can be selected to be substantially equal to a curvature of the wavefront of the resulting electromagnetic signal received by the array 20.

The receiving device 4 also includes a demodulator module 21 (DEM) connected to the array 20 and the processing module 5. Particularly, in accordance with the described example, the demodulator module 21 can include at least an IFDM (Inverse FDM) demodulator.

The processing module 5 can be structured to process the demodulated signals obtained from the array 20 by applying a processing algorithm to provide parameters representing measures of the backscattered field in term of different characteristics such as attenuation, polarization, resonances and interferences.

Particularly, the processing module 5 is configured to determine the presence of nulls or minima or values below a threshold in the scattering field as associated with the presence of tissue anomalies. The processing unit 5 allows running algorithms to localize the scattering nulls or minima in the pattern and it provides an indication to the user (as an example, on the display 16) who is informed that the probe antenna device 2 is in contact with a region of the patient's body wherein there is an in-homogeneity such as tumor tissues.

Examples of processing algorithms employable by the processing module 5 are described in the document EP-A-2465428 under the chapters "Examples of processing algorithms" and "Example of practical selection of signatures", herein enclosed as reference.

The array fixed on a curved supporting plate 23 of figure 3b having the above described selected curvature shows the advantage that substantially more than one antenna of the array 20 is capable of detect a null or minima when in presence of a tissue anomaly.

The controller module 14 is also configured to control the operation of the receiving module 4 via the link 15.

In accordance with a preferred but not limited embodiment of the device of the invention referring to the diagnosis of prostatic tumor, the plate reflector 3 has a length of 150-300 cm and a width of 60-150 cm, the probe antenna device 2 is positioned on the saddle shaped support 6 with respect to the reflector 3 at a height of 70-100 cm, and the array 20 is positioned with respect to the reflector 3 to a minimum height of 50-80 cm.

In operation, the incident electromagnetic signal radiated by the probe antenna device 1 irradiates the tissue of the patient who is sat on the external case 11 and part of this isotropic radiated signal reaches the reflector 3. A resulting backscattered signal S_{BS} emerges from the patient tissue and interferes with a reflected signal S_{R} reflected by the reflector 3: so producing an interference resulting signal S_{IN} which reaches the array 20.

Particularly, the reflectivity R of the reflector 3 is calibrated to simulate a mirror of the same signal from the antenna probe device 2 when in presence of a tumor tissue that enhances the nulls at the receiving antennas R1 and R2 only when the antenna probe device 2 is actually coupled with a tumor tissue, i.e. as the resulting backscattered signal S_{BS} and the reflected signal S_{R} mutually cancel only in this case, and interference null is so enhanced. The interference resulting signal is received and demodulated by the receiving device 4 and its null and minima, if any, are detected by the processing module 5, which can also show the results on the display 16.

It has been observed that the reflected signal S_{R} produced by the reflector 3 allows to improve the detectability of the nulls and minima.

The probe antenna device 2 is moved while the patient is sitting on the saddle shaped support 6 with the probe antenna device 2 in close contact with the patient body (e.g., low buttocks and perineum) so as to guarantee near-field coupling with tissues. Any movement of the probe antenna device 2 is controlled by the controller 14 over the link 15; received signals are processed by the processing module 5 to be prepared for displaying on the display 16 of clinical diagnosis. Moreover, it's to be noted that the sliding of the probe antenna device 2 is carried out by the clinician via the controller module 14 which also can report both the positions assumed by the antenna radiating element 12 and the measurements obtained from the array 20, so as to guarantee the repetition of the diagnostic test over multiple diagnostic sessions separated several weeks one another to diagnoses any change in tissues or the evolution of disease. In accordance with another example, the antenna radiating element 12 can be moved along the external case 11 by the clinician and without any motor.

Particularly, independently from the use of a motor, the processing module 5 can be configured to map the position of the probe antenna device 2 obtained from the control unit 14 with the corresponding nulls or minima. A processing method in the processing module 5 pairs the spatial positions of the nulls (or minima) at the array 20 with the position of the probe antenna device 2 moved purposely to localize the tumor at small-scale from multiple views.

Moreover, the Applicant observes that the described apparatus allows reducing the coupling between the clinician holding the antenna probe device 2 probe itself that might generate false nulls or minima with erroneous diagnosis. It's to be observed that seat means 6 can be configured to be comfortable enough to guarantee that the patient does not feel the uncomfortably of the still position, since accommodating the patient size and shape in order to preserve the same position in space of the anatomic part under investigation, i.e. in the concept that for the diagnostic usage each patient can be seen as composed of the anatomic part under diagnosis "surrounded by the patient's body".

### List of references employed in the figures:

- tissue anomaly detection apparatus 1000
- patient positioning structure 1
- probe antenna device 2
- reflector 3
- receiving device 4
- processing module 5 (PROC)
- seat means 6 (saddle shaped support)
- leg elements 7
- base structure 8
- footrest element 9
- support aid element 10
- external case 11
- antenna radiating element 12
- motor MT
- mechanical coupling module MC.
- internal case 13
- controller module 14
- wired or wireless link 15
- display 16 (DISP)
- a probe controller 17 (CNT)
- electromagnetic wave source 18 (WS)
- transmitter modulator 19 (M-TX)
- array 20
- receiving antennas R1 and R2
- demodulator module 21
- vertical flat support plane 22
- curved support plate 23

## Claims

1. A tissue anomaly detection apparatus (1000), comprising:
a patient positioning structure (1) configured to allow the patient assuming a detection position;
a probe antenna device (2) structured to radiate a generated radio-frequency signal to irradiate a patient tissue in near-field conditions so as to produce a first resulting radio-frequency signal (S_{BS}) ;
**characterised in that** it further includes:
a reflector (3) positioned so as to reflect at least part of the generated radio-frequency signal which has not irradiated the patient tissue and generate a reflected signal (S_{R});
a receiving device (4) structured to receive the first resulting signal and the reflected signal and provide corresponding received data;
a processing module (5) structured to process the received data to provide an information indicating detection of an anomaly in said tissue.

2. The apparatus (1000) of claim 1, wherein:
the receiving device (4) is configured to produce the received data from a second radio-frequency resulting signal (S_{IN}) corresponding to an interference of the reflected signal (S_{R}) and the first resulting signal (S_{BS}).

3. The apparatus (1000) of claim 1, wherein:
the processing module (5) is structured to:
analyse from the received data an electrical field power scattering pattern expressed versus at least one of the following parameters: at least one position of the antenna probe device (2), at least one position of the receiving device (4) and at least one frequency; and
detect said anomaly associated with nulls or minima in said pattern.

4. The apparatus (1000) of claim 1, wherein the reflector shows a reflectivity included in a range of 0.6 to 1.0.

5. The apparatus (1000) of claim 4, wherein the reflector (3) is a horizontal plate and the antenna probe device (2) is positioned between patient irradiated tissues and the horizontal plate.

6. The apparatus (1000) of claim 1, wherein:
the patient supporting structure (1) includes a supporting frame configured to support the probe antenna device (2).

7. The apparatus (1000) of claim 6, further including:
a motor (19) mechanically coupled to the probe antenna device (2) to cause a motion of the probe antenna device.

8. The apparatus (1000) of claim 7, wherein the antenna probe device includes:
an antenna radiating element (12);
an external case (11) housing the antenna radiating element;
and wherein the motor (19) mechanically coupled to the antenna radiating element (12) is structured to cause a sliding motion of the antenna radiating element in the external case (11).

9. The apparatus (1000) of claim 1, wherein the patient positioning structure (1) comprises:
seat means (1) adapted to accommodate the patient according to anatomy of said patient; and
wherein the antenna probe device (2) is mechanically fixed to said seat means.

10. The apparatus (1000) of claims 8 and 9, wherein said seat means comprises a saddle shaped support (2) on which said patient is adapted to sit astride; the seat means being structured to allow the patient being in contact with the external case (11) of the antenna probe device (2).

11. The apparatus (1000) according at least one of the preceding claims, **characterized in that** said patient positioning structure (1) is made of dielectric materials without dielectric contrast with respect to the external case housing the antenna radiating element.

12. The apparatus (1000) of claim 1, **characterized in that** said receiving device (4) comprises an array of antennas (20).

13. The apparatus (1000) of claim 12, wherein said antennas of the array (20) are placed according to a flat trend (22).

14. The apparatus (1000) of claim 13, wherein said antennas of the array are placed according to a curved surface (23) defining a concavity facing towards said probe antenna device; a curvature of the curved surface being substantially equal to a curvature wavefront of the second radio-frequency resulting signal (S_{IN}).

15. The apparatus (1000) according to at least claim 3, wherein the probe antenna device is movable to assume a plurality of operating positions and the processing module (5) is configured to map said plurality of positions with corresponding nulls or minima in the power scattering versus frequency pattern.
